# EUROPEAN PATENT SPECIFICATION

(11) **EP 2 456 497 B1**
(45) Date of publication and mention of the grant of the patent: **31.12.2014**
(21) Application number: 10740575.5
(22) Date of filing: 21.07.2010
(51) Int. Cl.: A61M 25/00, A61M 25/06

(54) **FORCE-TRANSMITTING ELEMENT FOR USE IN MEDICAL CATHETERS**
KRAFTÜBERTRAGUNGSELEMENT ZUR VERWENDUNG IN MEDIZINISCHEN KATHETERN
ÉLÉMENT DE TRANSMISSION DE FORCE À UTILISER DANS DES CATHETERS MEDICAUX

(30) Priority: 21.07.2009 GB 0912665; 21.07.2009 US 227337 P
(43) Date of publication of application: 30.05.2012
(73) Proprietor: Angiomed GmbH & Co. Medizintechnik KG, 76227 Karlsruhe (DE)
(72) Inventor: WUEBBELING, Martin, 68161 Mannheim (DE); MAIR, Jutta, 76275 Ettlingen (DE)
(74) Representative: Hoffmann Eitle
(86) International application number: PCT/EP2010/060559
(87) International publication number: WO 2011/009884

(56) References cited:
- WO-A1-2004/047899
- WO-A1-2004/098692
- WO-A1-2010/015676
- WO-A2-2006/009588
- US-A1- 2004 267 162
- US-A1- 2007 112 331
- US-A1- 2008 147 170

## Description

### Field of the Invention

The present invention relates to a force-transmitting element, useful in the manufacture of medical catheters, especially those being components of stent delivery systems, and catheter introducers. The present invention provides such a force-transmitting element, together with a method of manufacture thereof. The present invention further provides an application of the force-transmitting element in a catheter stent delivery system, and also in a catheter introducer.

### Background Art

Medical catheters are well-known in the art for a variety of purposes which include the delivery and retrieval of fluids, the positioning of diagnostic, therapeutic and surgical elements, and the placement of surgical implants such as stents. Stents are implants delivered to a body cavity such as a blood vessel which among other functions provide structural support to the location in which the stent is deployed. Stents are well-known in the art, and an example may be found in published patent application WO 2005/053574. To be useful, medical catheters must be able to be cleanly inserted into the appropriate body cavity, must be able to be navigated through often tortuous body passages to a precise location, and must then responsively carry out their function.

Medical catheters are navigated by a force applied endwise to the catheter to longitudinally direct the movement of the catheter and, in some cases, torque applied about the axis of the catheter to rotate the catheter. While catheters may be guided in their path through the body by means such as a guide wire over which the catheter travels, it is of importance that the operator have good control over longitudinal and, in some cases, rotational motion. It is simultaneously of importance that the catheter be sufficiently flexible to avoid the imposition of undue stress on the cavities through which the catheter passes. Finally, it is of importance that the catheter have sufficient structural rigidity to accurately determine the positioning of the distal tip of the catheter in use. While various diagnostic arrangements can measure the position of the catheter in the body during catheter navigation and positioning, for example using x-ray measurements to locate dense metal elements in the catheter tip, it is important that the position of the catheter not intentionally vary or otherwise drift during use.

Some catheter applications require that the catheter withstand considerable endwise force during operation, which can cause the catheter to compress and thus the positioning of the catheter to become inaccurate. For example, in stent delivery systems, a stent is conventionally deployed from within a sheath by a combination of tension withdrawing the sheath and compression maintaining the position. Such an arrangement is disclosed in WO 2005/053574. Such operation leads to the requirement that some element of the catheter be subject to endwise compressive forces. If the catheter has more than insignificant endwise resilience, for example when the catheter shaft is of synthetic polymeric material, compression of the catheter will result in the position of the stent varying from its initial location during deployment by some proportion of the catheter length. To compensate for this inaccuracy, operators conventionally rely on their own judgement and experience to position the stent slightly distally of the location at which the stent is deployed. Then, as tension is imposed on the sheath to release the stent progressively, the catheter shaft under compression will suffer a reduction in its length, resulting in the stent itself moving a small distance proximally before even the most distal part of the stent starts to be released. By "taking up the slack" in this way, the stent is deployed more closely to the desired location, as observed for example by radiological medical imaging techniques. However, reliance on judgement or experience may not be sufficient or convenient for all scenarios, particularly when catheters of varying lengths are to be used, or when varying catheter constructions or materials are used. Further, manipulation of the catheter during the stent release operation to reposition the stent may be inaccurate and inconvenient for the operator. There is also an issue as to the extent that the catheter length can resile even while the stent is being released, once the distal end of the stent is out of the sheath and the peak compressive stress in the length of the catheter shaft subsides.

Other catheter applications include so-called catheter introducers, which are larger-diameter tubes used to more easily introduce to and remove from the body special-purpose catheters, typically transcutaneously into a large-diameter blood vessel e.g. in the Seldinger technique. Such catheter introducers provide a definite navigable pathway to a first location in the body, and enable special-purpose catheters to be rapidly and easily navigated to their location of action. Catheter introducers, therefore, should provide a pathway with good structural integrity and resistance to applied internal or external forces while presenting an external configuration that will avoid harm to body passages. For example, catheter introducers, in use, should present no or minimal ovality under applied forces to enable a catheter of matching outer diameter to be cleanly introduced. Further, the catheter introducer should enable an operator to cleanly and responsively navigate the introducer to the desired location, and should thus provide good tactile feedback to the operator as to obstructions and ease of travel.

US 2008/147170 A forms the basis for the two-part form of claim. WO 2004/098692 A and US 2007/0112331 A1 are useful in understanding the contribution to the art made by the claimed invention.

### Summary of the Invention

According to the present invention, there is provided a force-transmitting element for a medical catheter comprising a tube and a tubular sleeve, wherein the tube has formed through the wall thickness a plurality of axially-distributed slits formed so that the extent of each slit in the circumferential direction exceeds half of the circumference of the tube such that, when the tube is longitudinally flexed into a curve, the slits on the outside of the curve become relatively more opened and, when the element is subject to no external endwise compressive forces, the tube is retained by the tubular sleeve in a state of endwise compression such that the slits are relatively more closed than if the sleeve were absent.

Such an arrangement is able to provide a flexible structure that can resist changes in length due to endwise axial compression more effectively, thereby allowing a tip location to be more accurately determined and maintained in use. Such an arrangement is contrary to technical prejudice in the art of stent delivery systems, which assumes that the "taking up of the slack" by the operator before deploying the catheter is unavoidable. Further, the presence of the sleeve permits the tube to be more suitably used for the transfer of torque between its proximal and distal ends. Enhancing structural axial stiffness in this way permits thinner tube walls to be used than in a structure not exhibiting such intrinsic structural axial stiffness.

Preferred embodiments of the present invention relate to particular advantageous constructions and materials which enable the advantages of the present invention to be more clearly realised.

Such preferred constructions include the provision of the outer membrane as a liquid-impermeable membrane. This permits the force-transmitting element to be incorporated in a liquid-conveying catheter as a primary means of isolating a liquid in the catheter (such as a flushing liquid or contrast agent) from the environment external to the catheter.

One further preferred embodiment of the present invention is a catheter comprising the force-transmitting element of the present invention.

Such an embodiment provides a catheter suitable for use in a stent delivery system and able to resist variation in catheter length, and thus stent position, during stent deployment.

Another further preferred embodiment of the present invention is a catheter introducer comprising the force-transmitting element of the present invention.

Such an embodiment provides better tactile feedback and responsive control to an operator manipulating the catheter introducer.

The invention also provides a method of manufacturing the force transmitting element of the present invention comprising the steps of providing a tube; forming within the tube a plurality of axially-distributed slits formed so that the extent of the slit in the circumferential direction exceeds half of the circumference of the tube such that, when the tube is longitudinally flexed into a curve, the slits on the outside of the curve become relatively more opened; and providing to the tube a tubular sleeve to form the element, such that, when the element is subject to no external endwise compressive forces, the tube is retained in a state of endwise compression by the tubular sleeve such that the slits are relatively more closed than if the sleeve were absent.

### Brief Description of the Accompanying Drawings

To better understand the present invention, and to show how the same may be carried into effect, reference will be made, by way of example only, to the accompanying Drawings, in which:
Figure 1a shows an embodiment of the present invention under longitudinal tension, being a force-transmitting element having one particular configuration of circumferential slits;
Figure 1b shows another embodiment of the present invention under longitudinal tension, being a force-transmitting element having one particular configuration of inclined slits;
Figure 2 shows the effect of the compression by the external sleeve on one slit in the force transmitting element of Figure 1a;
Figure 3 shows an example of an over-the-wire stent deployment system of the type in which the present invention may be put into effect;
Figure 4 shows an embodiment of the present invention, being a stent delivery system incorporating the force-transmitting element of the present invention;
Figure 5 shows an example of a typical catheter introducer illustrating what is considered to be known in the art; and
Figure 6 shows an embodiment of the present invention, being a catheter introducer incorporating the force-transmitting element of the present invention.

### Detailed Description

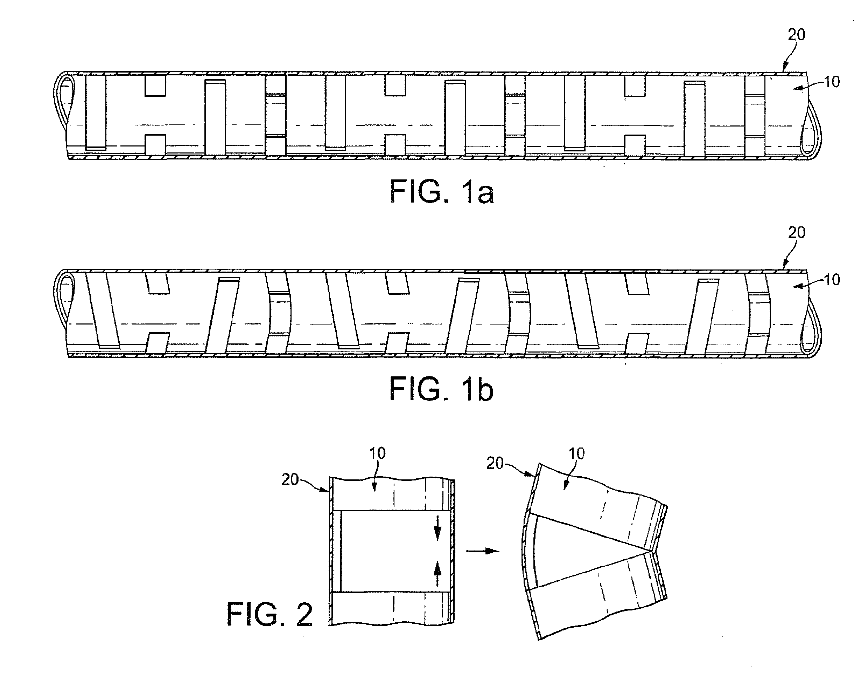

A force-transmitting element being an embodiment of the present invention is shown in Figure 1a, in a state of longitudinal tension. To form the element shown in Figure 1, a stainless steel tube 10 of axial length 100mm, wall thickness 60µm and inner diameter 6.5 French (where 1 French is defined to be 1/3mm) is formed. Such a bore is suitable for use in, for example, a catheter introducer. The skilled person will recognise that the bore may be varied to take into account the dimensional requirements of other catheter applications. Other materials are possible for the tube, including high-strength polymers and polymer composites. In other embodiments, other values of the axial length and wall thickness will be easily selected to determine the strength and flexibility properties required by the particular use to which the catheter is put.

A series of slits are cut spaced axially one from another into the tube extending to a depth (defined by the deepest-cut chord of the slit) of about 1.8 times the external radius of the tube; that is to say, the slits are cut about 90% through the diameter of the tube. The slits are formed as purely transverse circumferential cuts; geometrically, the chord of each slit has an angle with the local axis of the tube of 90 degrees. Axially neighbouring slits are cut at constant longitudinal intervals of 60µm, having a slit width of 20µm, and are related one to another by angular rotation of each slit relative to its neighbour by an angle of 90 degrees about the local axis of the tube. Geometrically, the chords of neighbouring slits have an angular relationship between them of 90 degrees. The slits are therefore cut in a staggered sequence about the tube, the apices of neighbouring slits forming a helix about the tube. Such a configuration provides increased flexibility and retains a significant proportion of the columnar strength of the original tube.

The angle of the chord of each slit relative to that of its neighbours is termed the helix angle, sometimes termed the step angle. Other helix angles are possible, preferentially helix angles of between 0 and 90 degrees are selected. The configuration having a helix angle of 90 degrees provides a good balance of flexibility and strength while minimising the tendency for the tube to coil.

Other embodiments have slits set at angles other than at 90 degrees to the local tube axis, and have other angular and spatial relationships between neighbouring slits. Figure 1b shows one alternative embodiment having slits inclined to the plane defined by the local axis of the tube with a helix angle of 90 degrees and equal spacing between the intersections of the slits with the diameter of the tube. The angle of inclination of the slits is exaggerated in Figure 1b to demonstrate the alternative to the embodiment of Figure 1b. In the embodiment of Figure 1b the slits are inclined in the same longitudinal direction from apex to chord, enabling the tube to be flexed in all directions equally. The slits, however, need not be cut in a plane, and embodiments having helically-curved slits are also envisioned. The embodiment of Figure 1a, however, is presently preferred due to ease of manufacture.

Variation is possible in the depth of the cut of the slits. Provided that the slit is cut more than half-way through the tube, so that the extent of the slit in the circumferential direction exceeds half of the circumference of the tube, the advantages of the present invention may be realised. Cutting to greater depths increases flexibility while reducing columnar strength. Cutting the slits leaves at least one so-called "bridge" of tube material adjoining neighboring tubular rings. Of course, cuts, such as longitudinal division into two or more bridges, may be made in the bridge to subdivide it, if enhanced flexibility at the cost of columnar strength is required.

variation is also possible in the width of the cut of the slits, and in the longitudinal pitch of the slits. Reducing the pitch will tend to increase flexibility, and vice versa. Any or all of these parameters may be varied along the length of the tube, in order to impart different strength and flexibility to different sections of the tube. Such longitudinal variation may be of importance to a designer who is seeking a highly flexible distal portion for ease of navigation within the body, while requiring a proximal portion having high columnar strength. Selection of such parameters by trial and experiment is well within the ambit of the skilled person in the art. Further, the slits may indeed have parallel walls, or may, for example, be tapered with the depth of cut.

The slits are cut by laser vaporisation of the wall of the tube in order to produce clean edges and to eliminate the requirement for disposing of waste materials. Other manufacturing processes, such as machining or moulding, may also be used, if preferred for the scale and material being used. Rapid prototyping techniques may be employed for small manufacturing runs or custom requirements. Electrochemical polishing techniques may conventionally be used to ensure that the slitted tube has a sufficiently smooth surface for the contemplated application.

Next, the tube is enclosed within a tubular sleeve 20 of, in the present embodiment, heat-shrink polymer. In the present embodiment, this sleeve is formed from polyamide tubing having wall thickness 20*µ*m and a tubular inner diameter of 7 French (2.33mm). In some embodiments, presently preferred, the sleeve is mechanically pre-tensioned longitudinally before or subsequent to its positioning around the tube. Such embodiments can give a greater degree of endwise compression to the tube, as will be described below.

The tube is then mechanically compressed endwise within the sleeve while located, for example, on a mandrel. The mandrel is sized to accommodate the tube on it, to ensure that the tube may be compressed linearly without distorting or buckling under the applied endwise compressive force. The force is increased until the apex of each slit, geometrically being the point on each slit furthest from the chord of that slit, closes. This change of state is shown in Figure 2, in an exaggerated fashion, as to the relative dimensions of slit and tube. Other embodiments having different degrees of apex closure can result from the application of different magnitudes of force, and the force required to achieve a different degree of apex closure will of course vary with the length of tube. It is entirely within the ambit of the skilled person to make such adjustments to meet the requirements of any use to which the force-transmitting element is put.

In the present embodiment, heat is then applied to the sleeve to cause it to shrink, and thus to snugly enclose and radially compress the already longitudinally-compressed tube. In the present embodiment, heat-shrinking is carried out at a temperature of 200°C. If the sleeve has been pre-tensioned longitudinally before heat-shrinking, an even greater degree of longitudinal compression may be applied to the tube by this step.

The sleeve is chosen such that the degree of radial compression achieved is sufficient to frictionally prevent the tube from longitudinally expanding once the external endwise force is released. It is at this stage of the process that, in other embodiments not already having a sleeve already fitted, the tubular sleeve may be applied, for example by spraying a polymeric coating, or by wrapping a tape helically about the tube.

In similar embodiments, the sleeve may be chosen so that the ends of the sleeve enclose the ends of the tube, thereby further constraining its expansion. If the compressive frictional interaction between inner surface of the sleeve and outer surface of the tube is sufficiently strong, however, such end-capping is not strictly necessary, as in the present embodiment. In other embodiments, the sleeve may be applied later by coating, lamination or moulding, rather than by heat-shrinking. It is sufficient to realise the invention that the sleeve be able to maintain structural integrity while applying a longitudinally compressive force to the tube. In some embodiments, application of adhesive to or roughening of either the sleeve inner surface or tube outer surface, or both, may be used to increase the ability of the sleeve to maintain the required degree of compression in the tube.

Contemplated further embodiments include the positioning of a pre-tensioned metal braiding about the tube which is subsequently welded at each end or at locations along the braiding such that, on release of tension, the braiding applies the necessary endwise compression to the tube. As an alternative to welding, or as an ancillary securing means, a heat-shrink polymer sleeve can be placed about the pre-tensioned braid enclosing the tube, which sleeve can be heat-shrunk to tightly compress the braid to the tube and thereby provide the engagement to transfer longitudinally-compressing forces from the braid to the tube.

Finally, the endwise compressive force is released; the tube is now held in a compressed state by the presence of the sleeve. Pre-tensioning the sleeve shows benefits here, as pre-tensioned sleeves exhibit reduced propensity to stretch after release of the endwise compressive force. Without pre-tensioned sleeves, the tube may slightly expand on release of compression and the apices of the slits may slightly open or further open, depending on the degree of initial compression. The force-transmitting element is now ready to be incorporated in the structure for which it has been created.

The force-transmitting element thus formed is resistant to longitudinal compression, as the slits are closed at their apices and thus will not easily close further. However, the force-transmitting element may easily be flexed, as deviation from a straight-tube configuration may be achieved by virtue of the slits on one or other side of the tube opening under flexion. The sleeve selected has sufficient resilience to permit this extension of one side or another, while tending to keep the structure as a whole under the longitudinal compression required.

This construction, being a composite slitted metal and polymer sleeve structure, is able to provide reduced wall thickness and comparable axial stiffness as compared with polymer tubes alone

Machining and other forming operations can be carried out to the tube either before or after the above assembly process. For example, it is conventional in catheter systems to form the distal tip of the catheter with an atraumatic structure to avert injury to the passages through which the catheter passes. Additionally, the proximal end of the tube is usually formed for connection to a hub unit or hand unit which remains outside the body and provides control and connectivity functions to the catheter.

Generally preferred when a metallic tube is used is that the tube is prepared in an annealed condition, in order to achieve an enhanced balance of the mechanical properties needed in use, including trackability, pushability, bendability and resistance to kinking and buckling, as well as capability to effectively transmit a torque along the length of the tube.

One exemplary application of the force-transmitting element is in stent deployment systems.

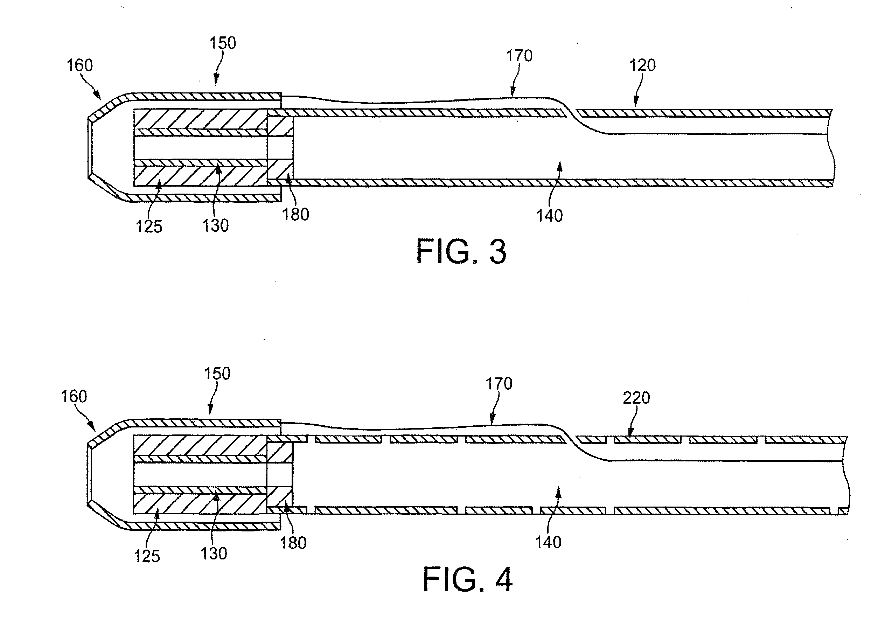

Figure 3 shows, for comparative purposes, an example of the distal end of a typical over-the-wire stent deployment system as known in the art. The entire stent deployment system has a proximal end and a distal end connected by a catheter shaft 120. The distal end carries a stent enclosed within a sheath, while the proximal end provides a hand-unit having liquid connections to the catheter and means for controlling deployment of the stent. Figure 3 is misleading in its scale, as the catheter shaft is longer than actually illustrated, and is of a length sufficient to locate the distal end in a desired body location while the proximal end remains at some distance outside the entry site, which may be, for example, in the leg.

The distal end shown in Figure 3 locates a self-expanding stent 125 in a state of radial compression on a stent support tube 130 having a guidewire lumen 140. The stent is held in radial compression by the sheath 150 which has an atraumatic tapered distal tip 160. The sheath is connected to a pull-wire 170 which is arranged to retract the sheath over the stent while under tension from the proximal end. The stent abuts a pusher annulus 180 located in the sheath proximal of the stent. The pusher annulus is arranged to provide longitudinal force to the stent as the sheath is withdrawn by means of the pull wire. Conventionally, the pusher annulus is coupled to the catheter shaft 120 which extends the length of the catheter to the proximal hand-unit and provides the necessary endwise compressive strength.

Other elements will naturally exist in particular constructions of stent delivery apparatus, such as multipart outer sleeves, specific lumen constructions and the like. However, these details will vary from application to application, and will be within the understanding of the skilled person to select and apply with regard to the purpose for which the apparatus is intended. The above-described features are sufficient to enable the skilled person to appreciate the context in which the invention may be put into effect.

In the stent delivery apparatus shown in Figure 4, being an embodiment of the present invention, the pusher annulus 180 is coupled to a force-transmitting element 220 as described above and as shown in Figure 1, instead of to the catheter shaft. The force-transmitting element is shown under tension for ease of visualisation. With no external forces acting, the tubular sleeve holds the slits closed, or near closed at their apices. In the apparatus of Figure 4, the force-transmitting element is attached to the pusher annulus by swaging. While the sheath 150 is withdrawn by means of the pull-wire 170, the stent 130 is compressed against the pusher annulus which transfers the compressive force to the force-transmitting element. As the force-transmitting element is already under longitudinal compression, the element will not significantly change in length under compression, and the requirement for pre-tensioning of the system by the operator prior to sheath retraction is reduced. Nevertheless, the force-transmitting element, by virtue of the slits, is able to flex more easily to conform to the curve of body passages than a homogeneous catheter shaft of comparable axial stiffness. As the force-transmitting element is sealed by the sleeve, a separate sealing tube is not necessary.

Whereas Figure 4 shows use of a force-transmitting element of the invention extending the full distance from the distal end of the system to the proximal end, this need not be the case in order to capture most of the benefit of the invention. For example, in a rapid-exchange stent delivery catheter system, much of the benefit of the invention is realised even when the distal end of the inventive force-transmitting element is proximal of the proximal guidewire exit port.

Another exemplary application of the force-transmitting element is for use in catheter introducers.

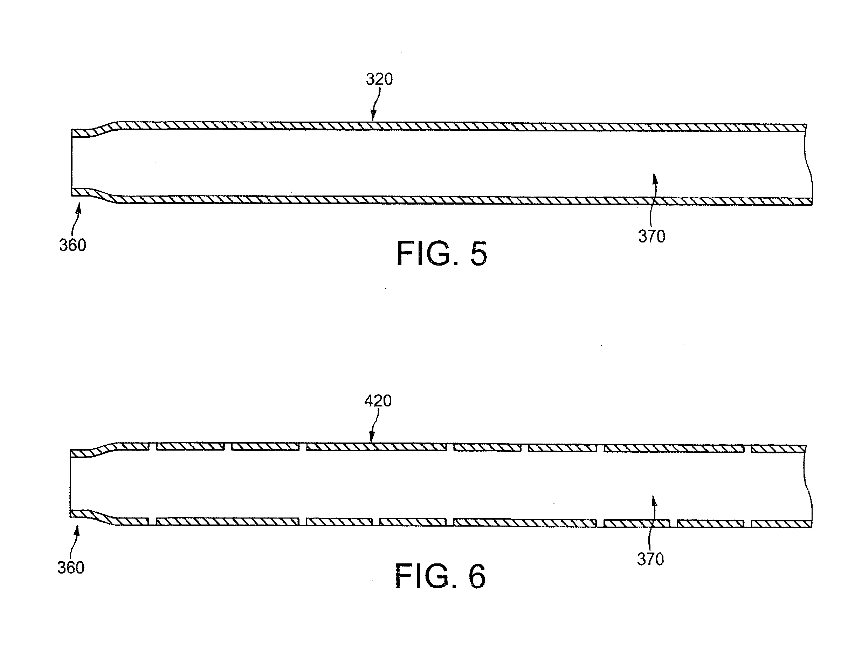

Figure 5 shows, for comparative purposes, an example of the distal end of a typical catheter introducer. The catheter introducer has a proximal end and a distal end joined by an introducer tube 320 having a central lumen 370. The distal end provides an atraumatic tip 360 with a central lumen being a continuation of that of the introducer tube 320 to allow a special-purpose catheter to be passed therethrough, while the proximal end provides a hand-unit having liquid connections to the introducer tube and conventional means for introducing a special-purpose catheter into the central lumen of the introducer tube. Figure 5 is misleading in its scale, as the catheter introducer aspect ratio is much smaller than illustrated, a typical diameters being 5mm or less, while a typical length might be 20cm. The displayed wall thickness is also heavily exaggerated to enable the reader to easily appreciate the structure of the introducer; typical wall thicknesses of known catheter introducers are on the order of 0.5mm wall thickness. The length is chosen to be sufficient to locate the distal end in a desired body location while the proximal end remains at some distance outside the entry site, which may be, for example, in the leg.

The catheter introducer is passed into the body through an incision and therefrom into a body cavity such as a blood vessel. The distal end of the catheter introducer is driven into the cavity by endwise compressive force, and may be navigated to that point by means of torsional rotation. The special-purpose catheter may then be inserted into the proximal bore of the catheter introducer and navigated to the deployment site easily and without undue concern as to the selection of the correct pathway or the possibility of damaging the walls of the body passages through which it passes.

In the catheter introducer shown in Figure 6, being an embodiment of the present invention, the introducer tube 420 is formed from the force-transmitting element described above and shown in Figure 1. As for Figure 5, dimensions are exaggerated. The force-transmitting element is shown under tension for ease of visualisation. With no external forces acting, the slits are normally closed or near closed at their apices, by virtue of the compression applied by the sleeve. Such a configuration allows the catheter to be navigated smoothly and responsively to the target location. As the force-transmitting element is already under longitudinal compression, the element will not significantly change in length under compression and will thus transmit contact and resistance sensations more directly to the hand of the operator. Nevertheless, the force-transmitting element, by virtue of the slits, is able to flex easily to conform to the curve of body passages. In embodiments of the present invention, for the same axial stiffness, the wall thickness can be significantly reduced. Alternatively, introducers of similar wall thickness to known introducers will have significantly enhanced axial stiffness.

The present invention is not limited to the presently-disclosed embodiments, but rather solely by the scope of the appended claims. The skilled reader will easily contemplate how embodiments of the force-transmitting element may be incorporated into other medical catheters and catheter introducers where dimensional stability and precise control of longitudinal translation and rotation are required. Such embodiments may not be herein explicitly described, but will nevertheless be clearly within the ambit of the skilled reader without undue experimentation and without the exercise of inventive skill.

## Claims

1. A force-transmitting element for a medical catheter comprising
a tube (10) and a tubular sleeve (20),
wherein the tube has formed through the wall thickness a plurality of axially-distributed slits formed so that the extent of each slit in the circumferential direction exceeds half of the circumference of the tube
such that, when the tube is longitudinally flexed into a curve, the slits on the outside of the curve become relatively more opened, **characterised in that**:
when the element is subject to no external endwise compressive forces, the tube is retained by the tubular sleeve in a state of endwise compression such that the slits are relatively more closed than if the sleeve were absent.

2. The force-transmitting element of claim 1, wherein the sleeve is liquid-impermeable.

3. The force-transmitting element of any preceding claim, wherein the slits are formed with a constant angular relationship between axially neighbouring slits.

4. The force-transmitting element of claim 3, wherein the constant angular relationship between axially neighbouring slits is defined as a fixed angle between the chords of neighbouring slits.

5. The force-transmitting element of claim 4, wherein the fixed angle is greater than or equal to 45 degrees and is less than or equal to 90 degrees.

6. The force-transmitting element of claim 4 or 5, wherein the fixed angle is 90 degrees.

7. The force-transmitting element of any preceding claim, wherein the slits are formed with a constant angular relationship to the axis of the tube.

8. The force-transmitting element of claim 7, wherein the constant angular relationship to the axis of the tube is defined as a fixed angle between the chord of the slit and the axis of the tube.

9. The force-transmitting element of claim 8, wherein the fixed angle is 90 degrees and the slits are thereby transverse circumferential slits.

10. The force-transmitting element of any one of claims 1 to 8, wherein the slits are helical slits.

11. A catheter comprising at least one lumen and the force-transmitting element according to any preceding claim, wherein the lumen enclosing the force-transmitting element and maintained in a fixed positional relationship.

12. A catheter according to claim 11, wherein the catheter is associated with a stent delivery system.

13. A catheter according to claim 12, wherein the force-transmitting element is arranged in the catheter to transmit endwise compressive forces arising during stent release.

14. A method of manufacturing a force-transmitting element for a medical catheter comprising the steps of
providing a tube;
forming within the tube a plurality of axially-distributed slits formed so that the extent of the slit in the circumferential direction exceeds half of the circumference of the tube
such that, when the tube is longitudinally flexed into a curve, the slits on the outside of the curve become relatively more opened;
and providing to the tube a tubular sleeve to form the element,
such that, when the element is subject to no external endwise compressive forces, the tube is retained in a state of endwise compression by the tubular sleeve such that the slits are relatively more closed than if the sleeve were absent.

15. A method of manufacturing a force-transmitting element for a medical catheter as recited in claim 14, further comprising a step of providing the force-transmitting element with the further features of any one of claims 2 to 13.

## Patentansprüche

1. Kraftübertragungselement für einen medizinischen Katheter mit
einem Rohr (10) und einer rohrförmigen Hülse (20), wobei das Rohr durch die Wanddicke eine Vielzahl axial verteilte Schlitze ausgebildet aufweist, die so ausgebildet sind, dass die Erstreckung von jedem Schlitz in der Umfangsrichtung die Hälfte des Umfangs des Rohrs überschreitet,
sodass, wenn das Rohr in Längsrichtung in eine Kurvenform gebogen wird, die Schlitze auf der Außenseite des Bogens relativ stärker geöffnet werden, **dadurch gekennzeichnet, dass**:
wenn das Element keinen am Ende wirkenden Druckkräften ausgesetzt ist, das Rohr durch die rohrförmige Hülse in einem Zustand eines vom Ende wirkenden Drucks gehalten wird, sodass die Schlitze relativ stärker geschlossen sind als wenn die rohrförmige Hülse nicht vorhanden wäre.

2. Kraftübertragungselement nach Anspruch 1, bei dem die Hülse flüssigkeitsundurchlässig ist.

3. Kraftübertragungselement nach einem der vorstehenden Ansprüche, bei dem die Schlitze mit einer konstanten Winkelbeziehung zwischen axial benachbarten Schlitzen ausgebildet sind.

4. Kraftübertragungselement nach Anspruch 3, bei dem die konstante Winkelbeziehung zwischen axial benachbarten Schlitzen als ein fester Winkel zwischen den Gurten benachbarter Schlitze definiert ist.

5. Kraftübertragungselement nach Anspruch 4, bei dem der feste Winkel größer oder gleich 45° ist und weniger oder gleich 90° ist.

6. Kraftübertragungselement nach Anspruch 4 oder 5, bei dem der feste Winkel 90° ist.

7. Kraftübertragungselement nach einem der vorstehenden Ansprüche, bei dem die Schlitze mit einer konstanten Winkelbeziehung zu der Achse des Rohrs ausgebildet sind.

8. Kraftübertragungselement nach Anspruch 7, bei dem die konstante Winkelbeziehung zu der Achse des Rohrs als ein fester Winkel zwischen dem Gurt des Schlitzes und der Achse des Rohrs definiert ist.

9. Kraftübertragungselement nach Anspruch 8, bei dem der feste Winkel 90° ist und die Schlitze dadurch querliegende Umfangsschlitze sind.

10. Kraftübertragungselement nach einem der Ansprüche 1 bis 8, bei dem die Schlitze schraubenförmige Schlitze sind.

11. Katheter mit mindestens einem Volumen und dem Kraftübertragungselement nach einem der vorstehenden Ansprüche, bei dem das Lumen das Kraftübertragungselement umgibt und in einer feststehenden Positionsbeziehung gehalten wird.

12. Katheter nach Anspruch 11, bei dem der Katheter mit einem Stentzuführsystem zusammenhängt.

13. Katheter nach Anspruch 12, bei dem das Kraftübertragungselement in dem Katheter angeordnet ist, um vom Ende wirkende Druckkräfte zu übertragen, die bei einer Stentfreigabe entstehen.

14. Verfahren zum Herstellen eines Kraftübertragungselements für einen medizinischen Katheter, das die Schritte umfasst
Bereitstellen eines Rohrs;
Ausbilden einer Vielzahl axial verteilter Schlitze in dem Rohr, die so ausgebildet sind, dass die Erstreckung der Schlitze in der Umfangsrichtung die Hälfte des Umfangs des Rohrs überschreitet,
sodass, wenn das Rohr in Längsrichtung in einer Kurvenform gebogen wird, die Schlitze auf der Außenseite des Bogens relativ stärker geöffnet werden;
und Bereitstellen einer rohrförmigen Hülse für das Rohr, um das Element auszubilden,
sodass, wenn das Element keinen äußeren vom Ende wirkenden Kräften ausgesetzt ist, das Rohr in einem Zustand vom Ende wirkenden Drucks durch die rohrförmige Hülse gehalten wird, sodass die Schlitze relativ stärker geschlossen sind als wenn die Hülse nicht vorhanden wäre.

15. Verfahren zum Herstellen eines Kraftübertragungselements für einen medizinischen Katheter nach Anspruch 14, ferner mit einem Schritt des Bereitstellens des Kraftübertragungselements mit den weiteren Merkmalen von einem der Ansprüche 2 bis 13.

## Revendications

1. Elément de transmission de force pour un cathéter médical, comprenant :
un tube (10) et un manchon tubulaire (20),
dans lequel le tube a, formées à travers l'épaisseur de la paroi, une pluralité de fentes réparties axialement, formées de sorte que l'étendue de chaque fente, dans la direction circonférentielle, dépasse la moitié de la circonférence du tube,
de sorte que, lorsque le tube est longitudinalement fléchi en une courbe, les fentes à l'extérieur de la courbe, sont relativement plus ouvertes, **caractérisé en ce que**:
lorsque l'élément n'est soumis à aucune force de compression externe entre les extrémités, le tube est retenu par le manchon tubulaire dans un état de compression entre les extrémités de sorte que les fentes sont relativement plus fermées que si le manchon était absent.

2. Elément de transmission de force selon la revendication 1, dans lequel le manchon est imperméable au liquide.

3. Elément de transmission de force selon l'une quelconque des revendications précédentes, dans lequel les fentes sont formées avec une relation angulaire constante entre des fentes axialement voisines.

4. Elément de transmission de force selon la revendication 3, dans lequel la relation angulaire constante entre des fentes axialement voisines est définie comme un angle fixe entre les cordes des fentes voisines.

5. Elément de transmission de force selon la revendication 4, dans lequel l'angle fixe est supérieur ou égal à 45 degrés et est inférieur ou égal à 90 degrés.

6. Elément de transmission de force selon la revendication 4 ou 5, dans lequel l'angle fixe est de 90 degrés.

7. Elément de transmission de force selon l'une quelconque des revendications précédentes, dans lequel les fentes sont formées avec une relation angulaire constante par rapport à l'axe du tube.

8. Elément de transmission de force selon la revendication 7, dans lequel la relation angulaire constante par rapport à l'axe du tube est définie comme un angle fixe entre la corde de la fente et l'axe du tube.

9. Elément de transmission de force selon la revendication 8, dans lequel l'angle fixe est de 90 degrés et les fentes sont ainsi des fentes circonférentielles transversales.

10. Elément de transmission de force selon l'une quelconque des revendications 1 à 8, dans lequel les fentes sont des fentes hélicoïdales.

11. Cathéter comprenant au moins une lumière et l'élément de transmission de force selon l'une quelconque des revendications précédentes, dans lequel la lumière entoure l'élément de transmission de force et est maintenue dans une relation positionnelle fixe.

12. Cathéter selon la revendication 11, dans lequel le cathéter est associé à un système de pose de stent.

13. Cathéter selon la revendication 12, dans lequel l'élément de transmission de force est agencé dans le cathéter pour transmettre des forces de compression entre les extrémités qui se produisent pendant la libération du stent.

14. Procédé pour fabriquer un élément de transmission de force pour un cathéter médical, comprenant les étapes consistant à :
prévoir un tube ;
former à l'intérieur du tube, une pluralité de fentes réparties axialement, formées de sorte que l'étendue de la fente dans la direction circonférentielle dépasse la moitié de la circonférence du tube,
de sorte que, lorsque le tube est longitudinalement fléchi en une courbe, les fentes à l'extérieur de la courbe sont relativement plus ouvertes ;
et prévoir sur le tube, un manchon tubulaire, afin de former l'élément,
de sorte que, lorsque l'élément n'est soumis à aucune force de compression externe, le tube est retenu dans un état de compression entre les extrémités par le manchon tubulaire de sorte que les fentes sont relativement plus fermées que si le manchon était absent.

15. Procédé pour fabriquer un élément de transmission de force pour un cathéter médical selon la revendication 14, comprenant en outre une étape consistant à doter l'élément de transmission de force des autres caractéristiques selon l'une quelconque des revendications 2 à 13.
